Europäisches Patentamt

European Patent Office

Office européen des brevets

(11) Publication number: **0 393 219**
**A1**

## (12) EUROPEAN PATENT APPLICATION

(21) Application number: **89107087.2**

(51) Int. Cl.⁵: **A46B 15/00, A46B 7/04**

(22) Date of filing: **20.04.89**

(43) Date of publication of application:
**24.10.90 Bulletin 90/43**

(84) Designated Contracting States:
**AT BE CH DE ES FR GB GR IT LI LU NL SE**

(71) Applicant: **Lin, Tong Liang**
**No. 6, Lane 55 Tao Yuan Street**
**Changhua(TW)**

(72) Inventor: **Lin, Tong Liang**
**No. 6, Lane 55 Tao Yuan Street**
**Changhua(TW)**

(74) Representative: **Helms, Joachim, Dipl.-Ing.**
**Bothmerstrasse 14**
**D-8000 München 19(DE)**

(54) **Tooth brush with tongue scaler.**

(57) A toothbrush consists of a handle (2) and brush head (2) to which are removably connected, respectively, a tongue scraper (5) and a bristle carrier (3). The tongue scraper (5) is insertable in a recess (210) of the handle (2), and can be removed by pulling the pull knob (53) at the rear end of the scraper (5). The bristle carrier (3) fits into a recess (211) through the top of the brush head (21) and is supported by lugs (34, 31) on either side. A cover plate (4) slides over the carrier (3) engaging both it and the bursh head (21) to lock the former in place.

Fig. 1

EP 0 393 219 A1

## TOOTH BRUSH WITH TONGUE SCALER

The present invention relates to a tooth brush with tongue scaler, which is comprised of a brush head integrally connected with a brush handle, a bristle holder, a positioning cover plate, and a tongue scaler, wherein the brush head is having a receiving trough for setting therein of the bristle holder; the cover plate is connected with the bristle holder by means of setting the hoses of the bristle holder in the positioning recesses of the cover plate, and connected to the brush head by setting the projecting end of the sliding rod of the cover plate in the recess of the hole of the stop wall of the brush head; and the tongue scaler is movably connected with the brush handle by setting the noses of the pull knob of the tongue scaler in the retaining grooves of the brush handle.

Regular tooth brush is comprised of a brush handle with bristles planted on the front end of the brush handle. After a certain period of use, the bristles are easy to wear away or to get slit. If to keep using the slit tooth brush, it will be unable to thoroughly clean the teeth and may possibly do hurt to the gingiva. Therefore, when a tooth brush is wearing out, people normally will give it away to buy a new one. Howvere, to throw away a whole tooth brush is not economic. For cost saving's sake, a variety of brush head or bristle holder replaceable tooth brushes are produced to meet the demand. There are still problems existed in these brush head or bristle holder replaceable tooth brushes, as hereunder:

1. In the tooth brushes wherein the brush head is connected with brush handle by means of screw joint: Because resistance force is resulted from brushing of the tooth brush against the teeth and the gingiva, the brush head may work loose easily to become bad in performance.

2. In the tooth brushes wherein the bristle holder is connected with brush handle by means of slide-in joint: Because the tooth brush is used to brush the teeth along any directions, the motion of the tooth brush along the sliding way of the bristle holder drives the bristle holder rub against the resistance force resulted from the teeth to easily make the bristle holder become loosening.

As we know, human tongue is a precicion and important organ in the ingestion of food, the perception of taste, and the articulation of speech sounds, and which is comdprised of lingual papillae. However, dirts may reside on the tongue to form into one layer of fur which may produce an oder smell, make people feel dry and bitter and interfere with the normal functioning of the tongue.

Some people who care more about the health of one's tongue may use a brush or a spoon to clear off the fur from one's tongue before of after brushing the teeth. However, the bristles of a tooth brush can not thoroughly scale the fur from the tongue due to the gaps among the bristles, and the spoon is very inconvenient to operate or easy to hurt one's tongue during the operation (smaller spoon has relatively a smaller side edge and is not suitable for scaling the fur from the tongue while larger spoon may occupy the whole cavity of the mouth to interfere with the operation.

In view of said problems, the present invention is designed to provide such a tooth brush which is practical and economic for use to clean the teeth as well as the tongue.

The present invention is related to a tooth brush with tongue scaler, which can be used for brushing the teeth and for scaling the fur of human tongue concomitantly.

The main object of the present invention is to provide a tooth brush with tongue scaler, wherein the bristle holder is replaceable, in case it becomes slit and worn away after a certain period of application, to permit the present invention to be reusable.

Another object of the present invention is to provide a tooth brush with tongue scaler, wherein the brush head is having one pair of guide rails set at the both lateral sides and the bristle holder is having a sliding way set on the top at the middle so as to allow easy connection of the positioning cover plate by letting the stepped tracks and the sliding rod of the cover plate slide along the guide rails of the brush head and the sliding way of the bristleholder respectively.

A yet further object of the present invention is to provide a tooth brush with tongue scaler, wherein the movable tongue scaler which is set in the brush handle may be detached from the brush handle for independent application of partly pulled out of the brush handle for scaling the fur from human tongue.

In order that the invention may be better understood, a preferred embodiment will now be described, by way of example only, with reference to the accompanying diagrammatic drawings, in which:-

Figure 1 is a perspective fragmentary view of the present invention;

Figure 2 is a perspective assembly view of the present invention;

Figure 3 illustrates the positioning of the positioning cover plate;

Figure 4 illustrates a condition of the present invention wherein the tongue scaler is pulled out the brush handle; and

Figure 5 illustrates the application of the tongue scaler.

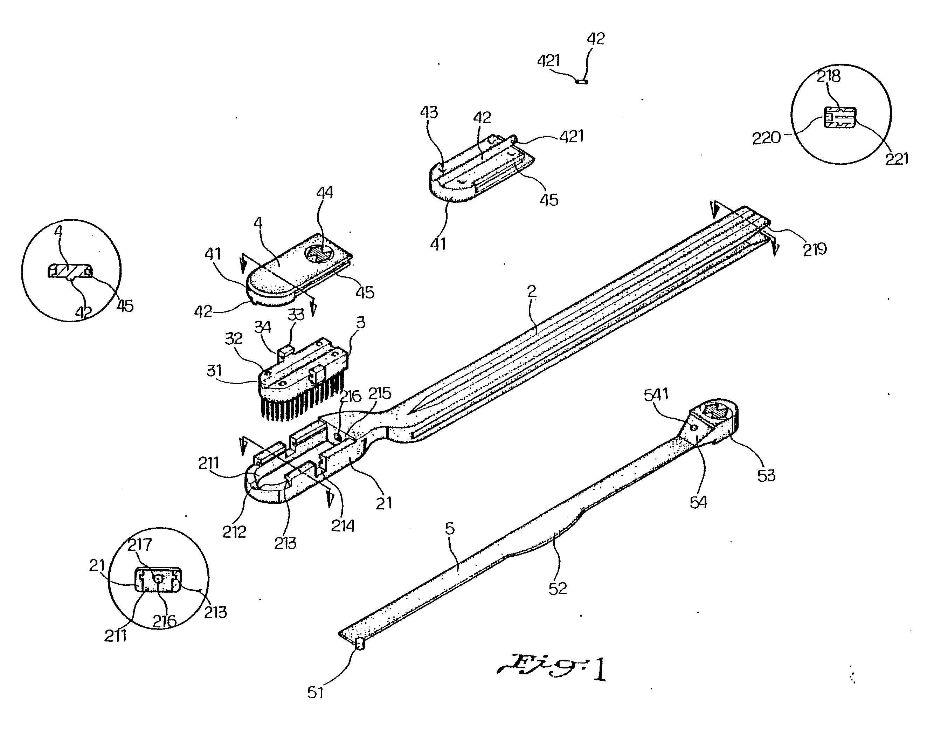

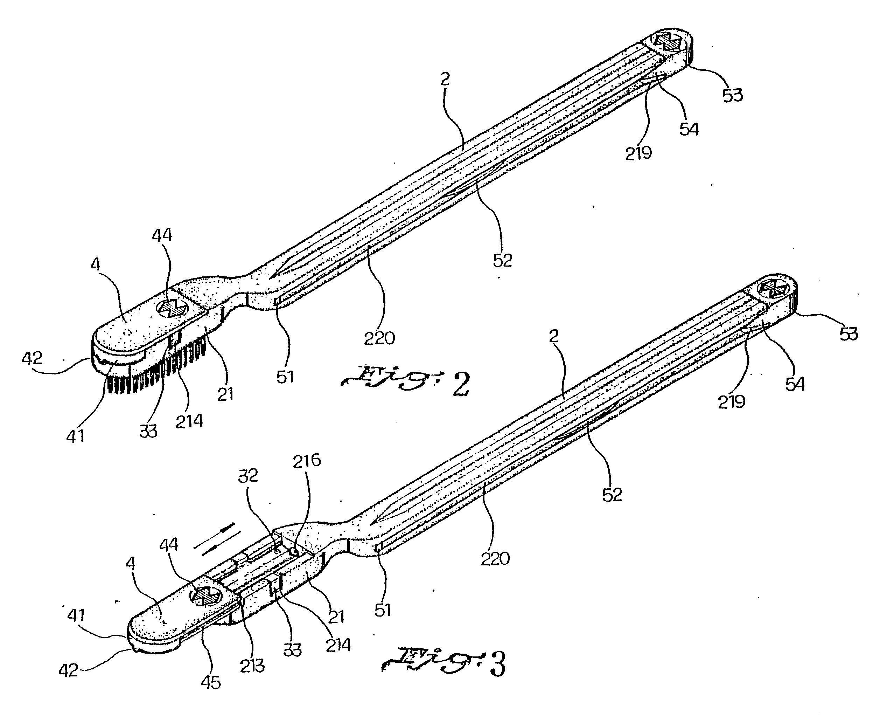

Referring to figures 1 and 2, a brush handle 2 is integrally connected with a brush head 21 which brush head 21 is having a receiving trough 211 pierced therethrough; a sliding way 212 made at the front edge of the receiving trough 211; one pair of guide rails 213 made at both sides respectively; one pair of notches 214 respectively made on both lateral sides; a raised stop wall 215 set at the inner end, which stop wall 215 is having hole 216 made opposite to the sliding way 212 with a recess 217 set therein. The brush handle 2 is having a v-shaped opening 219 made at the rear end; two retaining grooves 218 made at the upper and lower center of the bottom end respectively; and an elongated and stepped slot made at one lateral side, which stepped slot is comprised of an elongated outer slot 220 and an elongated inner slot 221. A bristle holder 3 is having a longitudinally disposed elongated sliding way 31 made on its top surface, one and more pairs of symmetric noses 32 bilaterally made by the sliding way, and two retainer lugs 33 respectively made on both lateral sides of the bristle holder 3 by the sliding way 31 with a guide groove 34 respectively pierced through each retainer lug 33 at the inner side, such that the bristle holder 3 will be firmly connected with the brush head 21 when the retainer lugs 33 of the bristle holder 3 are respectively set in the notches 214. A positioning cover plate 4 is having a stopper 41 made at the front, an elongated sliding rod 42 made on the bottom surface along axial direction having a projecting end 421 protruding beyond the rear end of the cover plate 4, one and more pairs of positioning recesses 43 and one pair of stepped tracks 45 respectively made at both sides. When in assembly, the sliding rod 42 is set in the sliding way 212 of the brush head 21 of the brush 2, and the positioning cover plate 4 is pushed with a finger through the antiskid stripes 44, which is made on the top surface of the positioning cover plate 4, to slide forward until the front stopper 41 is stopped by the bilateral front edges of the brush head 21 so as to let the positioning noses 32 be respectively set in the positioning recesses 43, to let the projecting end 421 of the sliding rod 42 be set in the recess 217 of the hole 216 of the stop wall 215. By means of this arrangement, resistance from friction force is minimized, and the bristle holder 3 is firmly retained by the positioning cover plate 4 in the receiving trough 211 of the brush head 21. An elongated flat tongue scaler 5 is provided for scaling the fur from human tongue, comprising a retainer stub 51 set at the front edge, a curved side edge 52 made at the middle part at one lateral side, and a pull knob 53 integrally made at the other end opposite to the retainer stub 51, which pull knob 53 is having a V-shaped slop 54 with one pair of symmetric noses 541 made thereon at the upper and the lower side respectively. When in assembly, the tongue scaler 5 is set in the elongated inner slot 221 with the retainer stub 51 set in the elongated outer slot 220 of the handle 2, and with the two noses 541 of the V-shaped slope 54 respectively set in the retaining grooves 218 of the V-shaped opening 219, such that the tongue scaler 5 is firmly retained in the handle 2.

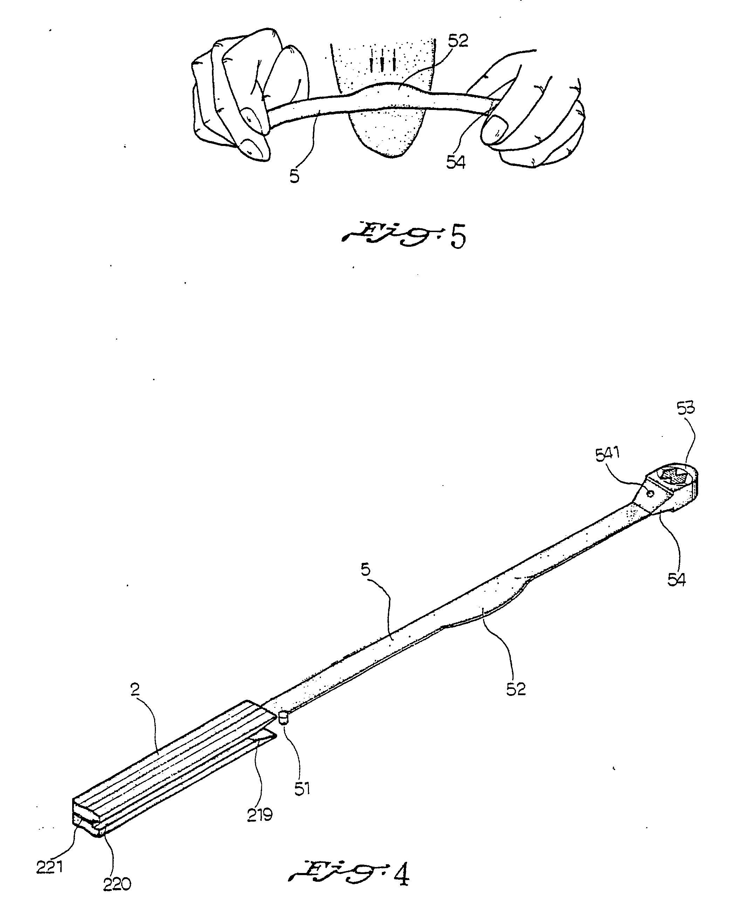

Referring to Figure 3, the bristle holder 3 is set in the receiving trough 211 with the retainer lugs 33 of the bristle holder 3 set in the notches 214 of the brush head 21, and the positioning cover plate 4 is pushed inward, by means of push force applied on the antiskid stripes 44, with the sliding rod 42 set in the sliding way 212 to let the bilateral stepped tracks 45 slide along the bilateral guide rails 213, and to let the sliding rod 42 slide along the sliding ways 212 and 31, so as to let sliding rod 42 insert into the hole 216 with the projecting end 421 set in the recess 217 of the hole 216 and to let the positioning noses 32 be respectively set in the positioning recesses 43 of the cover plate 4 upon connection thereto of the cover plate 4. When the bristle of the bristle holder 3 is wearing away and needs to be replaced, a push force is applied in a reverse way to break the positioning noses 32 away from the positioning recesses 43 and to separate the sliding rod 42 from the hole 216 of the handle 2 so as to let the cover plate 4 be removed from brush holder 3. The tongue scaler 5 is set in the elongated inner slot 221 with the front retainer stub 51 set in the elongated outer slot 220, which may be pulled out or set back along the axial direction by means of the operation of the pull knob 53 and which may be detached from the brush for use independently. When not in use, the tongue scaler 5 may be set inside the handle 2 with the Y-shaped slope 54 set in the V-shaped opening 219 of the brush handle 2, letting the noses 541 be set in the retaining grooves 218 respectively.

Referring to Figure 5, the tongue scaler 5 may be detached from the brush handle 2 or partly pulled out therefrom, and bent into an arch shape for use to clean off the fur of the tongue by means of reciprocating motion.

## Claims

A tooth brush with tongue scaler, including:
- a brush handle, being integrally connected with a brush head, having a V-shaped opening made at the rear end, two retaining grooves made at the upper and lower center of the rear end respectively, and an elongated and stepped slot made at one lateral side, said stepped slot being comprised of an elongated outer slot and an elongated inner slot, wherein said brush head is having a receiving trough pierced therethrough, a sliding way made at the front edge of said receiving trough, one pair of guide rails made at both sides respectively, one pair of notches respectively made on both lateral sides, a raised stop wall set at the inner end, said stop wall having hole made opposite to the sliding way with a recess set therein;

- a bristle holder having a longitudinally disposed elongated sliding way made on its top surface, one and more pairs of symmetric noses bilaterally made by both lateral sides of said sliding way, and two retainer lugs respectively made on both lateral sides of said bristle holder at the middle part with a guide groove respectively pierced through each of said two retainer lug at the inner side;

- a positioning cover plate having a stopper made at the front, a portion of antiskid stripes made on the top surface, an elongated sliding rod made on the bottom surface along axial direction comprising a projecting end protruding beyond the rear end of said cover plate, one and more pairs of positioning recesses and one pair of stepped tracks respectively made at both sides; and

- an elongated flat tongue scaler, comprising a retainer stub set at the front edge, a curved side edge made at the middle part at one lateral side, and a pull knob integrally made at the other end opposite to said retainer stub, said pull knob having a V-shaped slope with one pair of symmetric noses made thereon at the upper and the lower side respectively, wherein the tooth brush is characterized in that by means of the above-described arrangement, said bristle holder is set in said receiving trough with said retainer lugs of said bristle holder set in said notches of said brush head, to allow said positioning cover plate be pushed inward, by means of push force applied on said antiskid stripes, with said sliding rod set in said sliding way of said bristle holder to let said bilateral stepped tracks slide along said bilateral guide rails and to let said sliding rod slide along said sliding ways of said bristle holder and said brush head so as to let said sliding rod insert said hole of said stop wall with said projecting end set in said recess of said hole and to let said positioning noses be respectively set in said positioning recesses of said cover plate upon connection thereto of said cover plate, and said tongue scaler is set in said elongated inner slot with said retainer stub set in said elongated outer slot of said handle, and with said two noses of said V-shaped slope respectively set in said retaining grooves of said V-shaped opening to let said tongue scaler be firmly retained in said handle.

Fig.1

Fig. 2

Fig. 3

EP 0 393 219 A1

*Fig. 5*

*Fig. 4*

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-1 495 675 (COLT)<br>* Page 1, lines 36-62; figures 1-5 *<br>--- | 1 | A 46 B 15/00<br>A 46 B 7/04 |
| A | GB-A- 1 932 (COSBY)(A.D. 1910)<br>* Page 3, line 18 - page 4, line 39; figures 1-27 *<br>--- | 1 | |
| A | DE-C- 375 077 (GÖRLICH)<br>* Figures 1-4 *<br>----- | 1 | |

**TECHNICAL FIELDS SEARCHED (Int. Cl.5)**

A 46 B

The present search report has been drawn up for all claims

| Place of search | Date of completion of the search | Examiner |
|---|---|---|
| THE HAGUE | 09-11-1989 | ERNST R.T. |

EPO FORM 1503 03.82 (P0401)